(19) Europäisches Patentamt
European Patent Office
Office européen des brevets

(11) Veröffentlichungsnummer: **0 386 681 A1**

# (12) EUROPÄISCHE PATENTANMELDUNG

(21) Anmeldenummer: **90104215.0**

(22) Anmeldetag: **05.03.90**

(51) Int. Cl.5: **C07D 207/34, C07D 405/04, A01N 43/36**

Die Bezeichnung der Erfindung wurde geändert (Richtlinien für die Prüfung im EPA, A-III, 7.3).

(30) Priorität: **08.03.89 CH 865/89**

(43) Veröffentlichungstag der Anmeldung: **12.09.90 Patentblatt 90/37**

(84) Benannte Vertragsstaaten: **AT BE CH DE DK ES FR GB IT LI LU NL SE**

(71) Anmelder: **CIBA-GEIGY AG**
**Klybeckstrasse 141**
**CH-4002 Basel(CH)**

(72) Erfinder: **Sutter, Marius, Dr.**
**Klingental 5**
**CH-4058 Basel(CH)**

(74) Vertreter: **Zumstein, Fritz, Dr. et al**
**Bräuhausstrasse 4**
**D-8000 München 2(DE)**

## (54) 3-Aryl-4-cyano-pyrrol-Derivate, Verfahren zu ihrer Herstellung und diese enthaltende mikrobiozide Mittel.

(57) Neue 3-Aryl-4-cyanopyrrol-Derivate der allgemeinen Formel

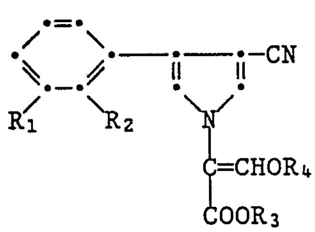

(I),

in welcher
$R_1$ und $R_2$ voneinander unabhängig unsubstituiertes oder halogensubstituiertes Alkyl, Halogen, Nitro oder Wasserstoff, oder
$R_1$ und $R_2$ zusammen $-OCF_2O-$, $R_3$ Alkyl, Cycloalkyl oder Benzyl und
$R_4$ Wasserstoff oder ein Alkali- oder Erdalkalimetall bedeutet.

Die neuen Wirkstoffe dienen der Bekämpfung schädlicher Mikroorganismen, vor allem phytopathogener Pilze. Sie können zusammen mit geeigneten Formulierungshilfsstoffen als Mittel eingesetzt werden und eignen sich gleichfalls zur Verhütung des Befalls von Kulturpflanzen durch schädliche Mikroorganismen.

EP 0 386 681 A1

## Mikrobizide Mittel

Die vorliegende Erfindung betrifft neue substituierte 3-Aryl-4-cyanopyrrol-Derivate, deren Herstellung, sowie mikrobizide Mittel, die als Wirkstoff mindestens eine dieser Verbindungen enthalten. Die Erfindung betrifft ferner die Herstellung der genannten Mittel und die Verwendung der neuen Wirkstoffe und Mittel zur Bekämpfung schädlicher Mikroorganismen, insbesondere pflanzenschädigender Pilze.

Die erfindungsgemässen Verbindungen haben die allgemeine Formel I

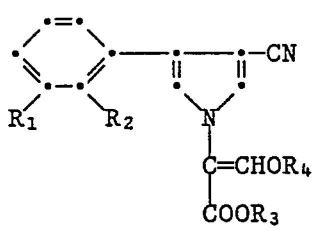

(I),

in welcher

$R_1$ und $R_2$ voneinander unabhängig unsubstituiertes oder halogensubstituiertes $C_1$-$C_8$-Alkyl, ferner Halogen, Nitro oder Wasserstoff, oder $R_1$ und $R_2$ zusammen $-OCF_2O-$,

$R_3$ $C_1$-$C_8$-Alkyl, Cycloalkyl oder Benzyl und

$R_4$ Wasserstoff oder ein Alkali- oder Erdalkalimetall bedeuten.

Aufgrund ihrer ausgeprägten mikrobiziden Wirkung sind diejenigen Verbindungen der Formel I bevorzugt, bei welchen $R_4$ Wasserstoff bedeutet. Von diesen Verbindungen sind jene insbesondere bevorzugt, bei welchen $R_1$ und $R_2$ voneinander unabhängig unsubstituiertes oder halogensubstituiertes $C_1$-$C_4$-Alkyl, Halogen, Nitro oder Wasserstoff, oder $R_1$ und $R_2$ zusammen $-OCF_2O-$ und $R_3$ $C_1$-$C_6$-Alkyl oder Benzyl bedeuten.

Unter dem Begriff Alkyl sind je nach der Zahl der angegebenen Kohlenstoffatome beispielsweise folgende Gruppen zu verstehen: Methyl, Ethyl, Propyl, Butyl, Pentyl, Hexyl, Octyl, usw., sowie ihre Isomeren, wie z.B. Isopropyl, sek.Butyl, Isobutyl, tert.-Butyl, Isopentyl, Isohexyl, usw.. Haloalkyl steht für einen einfach bis perhalogenierten Alkylsubstituenten, wie z.B. $CH_2Cl$, $CHCl_2$, $CCl_3$, $CH_2Br$, $CHBr_2$, $CBr_3$, $CH_2F$, $CHF_2$, $CF_3$, $CCl_2F$, $CCl_2$-$CHCl_2$, $CH_2CH_2F$, $CJ_3$ usw. Unter Halogen soll hier und im folgenden Fluor, Chlor, Brom oder Jod, vorzugsweise Fluor, Chlor oder Brom, verstanden werden.

Unter Cycloalkyl sind Cyclopropyl, Cyclobutyl, Cyclopentyl, Cyclohexyl oder Cycloheptyl zu verstehen.

Von den vorgenannten Verbindungen sind solche besonders bevorzugt bei denen $R_1$ und $R_2$ voneinander unabhängig Methyl, Trifluormethyl, Chlor, Brom oder Wasserstoff, oder $R_1$ und $R_2$ zusammen $-OCF_2O-$ und $R_3$ Methyl, Aethyl, n-Hexyl oder Benzyl bedeuten.

Hervorzuheben sind davon jene Verbindungen, bei welchen $R_3$ Methyl oder Aethyl bedeutet.

Hiervor sind als Einzelverbindungen wegen ihrer hervorragenden fungiziden Eigenschaften der 2-[3-(2,2-Difluorbenzodioxol-4-yl)-4-cyanopyrrol-1-yl]-3-hydroxyacrylsäuremethylester und der 2-[3-(2,3-Dichlorphenyl)-4-cyano-pyrrol-1-yl]-3-hydroxyacrylsäuremethylester zu nennen.

Die Verbindungen der Formel I sind unter Normalbedingungen stabile Oele, Harze oder überwiegend kristalline Feststoffe, die sich durch äusserst wertvolle mikrobizide Eigenschaften auszeichnen. Sie lassen sich beispielsweise auf dem Agrarsektor oder verwandten Gebieten präventiv und kurativ zur Bekämpfung pflanzenschädigender Mikroorganismen einsetzen. Die erfindungsgemässen Wirkstoffe der Formel I zeichnen sich in weiten Anwendungskonzentrationen durch eine hohe fungizide Aktivität und problemlose Anwendung aus.

Die Verbindungen der Formel I werden erfindungsgemäss hergestellt, indem man eine Verbindung der Formel II in Gegenwart einer Base mit einer Verbindung der Formel III oder mit einem anderen Formylierungsreagens

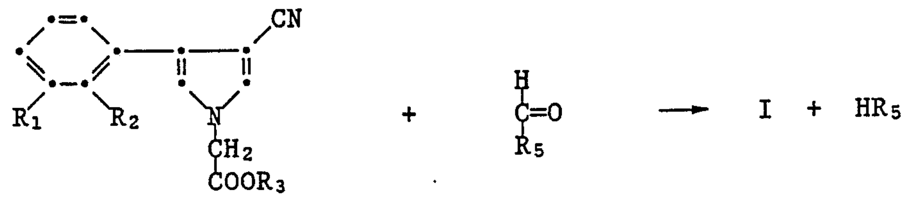

II III

umsetzt, wobei die Reste $R_1$ - $R_3$ die unter Formel I angegebene Bedeutung haben und $R_5$ einen Alkoxy- oder einen Dialkylaminorest bedeutet.

Das nach diesem Verfahren hergestellte Reaktionsprodukt kann als Enolatsalz, wobei $R_4$ in der Formel I ein Alkali- oder Erdalkalimetall bedeutet, oder als freies Enol, wobei $R_4$ Wasserstoff bedeutet, vorliegen. Aus den Enolatsalzen können die Enole z.B. durch die Zugabe von Säuren, wie beispielsweise wässrige Salzsäure freigesetzt und isoliert werden.

Die Umsetzung wird in einem Temperaturbereich von -30 bis 150° C, vorzugsweise 20-80° C in einem geeigneten Lösungsmittel durchgeführt. Dabei werden als Lösungsmittel Tetrahydrofuran, Aether und Acetonitril bevorzugt. Ebenfalls bevorzugt werden solche Lösungsmittel, welche gleichwohl als Formylierungsmittel dienen, wie beispielsweise die Ester der Ameisensäure.

Als geeignete Basen können Alkali- oder Erdalkalihydride, -hydroxide und -alkoholate wie z.B. NaH, KOH und $NaOR_3$ genannt werden.

Geeignete Formylierungsreagenzien ausser den Verbindungen der Formel III sind aus der Literatur bekannt (G.A. Olah et. al., Chemical Reviews, 87, 67 (1987). Die Herstellung der Zwischenprodukte der Formel II erfolgt analog zu bekannten Kondensationsreaktionen durch Umsetzung des 3-Aryl-4-cyanopyrrols (IV) mit einem Halogenessigsäureester wie beispielsweise Bromessigsäuremethylester (V) in Gegenwart einer Base, wie beispielsweise $K_2CO_3$:

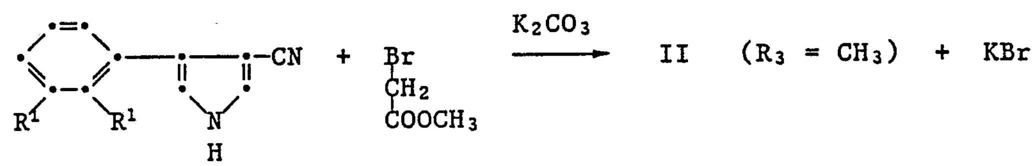


IV V

In der EP-182,737 werden zu den Verbindungen der Formel I vorliegender Erfindung strukturähnliche Mikrobizide beschrieben, die eine Propionsäureester-Gruppe am N-Atom des Pyrrols tragen.

Im Vergleich zu diesen bekannten N-substituierten Derivaten von 3-Phenyl-4-cyanopyrrolen, weisen die erfindungsgemässen Verbindungen eine sprunghaft erhöhte verbreiterte fungizide Aktivität auf.

Es wurde nun überraschend festgestellt, dass die erfindungsgemässen Verbindungen der Formel I ein sehr vorteilhaftes, die praktischen Bedürfnisse gut befriedigendes biozides Wirkungsspektrum gegen schädliche Mikroorganismen, insbesondere gegen phytopathogene Pilze und Bakterien, aufweisen. Sie besitzen sehr vorteilhafte kurative, systemische und insbesondere präventive Eigenschaften und lassen sich zum Schutz von zahlreichen Kulturpflanzen einsetzen. Mit dem Wirkstoff der Formel I können an Pflanzen oder an Pflanzenteilen (Früchte, Blüten, Laubwerk, Stengel, Knollen, Wurzeln) von unterschiedlichen Nutzkulturen die auftretenden Schädlinge eingedämmt oder vernichtet werden, wobei auch später zuwachsende Pflanzenteile vor phytopathogenen Mikroorganismen verschont bleiben.

Die erfindungsgemässen Wirkstoffe sind beispielsweise gegen die den folgenden Klassen angehörenden phytopathogenen Pilze wirksam: Ascomycetes z.B. Erysiphe, Sclerotinia, Fusarium, Monilinia, Helminthosporium; Basidiomycetes wie z.B. Puccinia, Tilletia, Rhizoctonia; sowie die der Klasse der Phycomycetes angehörenden Oomycetes wie Phytophthora. Als Pflanzenschutzmittel können die Verbindungen der Formel I mit besonders gutem Erfolg gegen wichtige Schadpilze aus der Familie der Fungi imperfecti eingesetzt werden, so z.B. gegen Cercospora, Pyricularia und vor allem gegen Botrytis. Botrytis spp. (B. Cinerea, B. allii) stellen mit der Grauschimmelfäule an Reben, Erdbeeren, Aepfeln, Zwiebeln und anderen Obst- und Gemüsesorten einen bedeutenden wirtschaftlichen Schadfaktor dar. Dabei weist insbesondere die Verbindung Nr. 1.1 aus Tabelle 1 ein breites Wirkungsspektrum auf. Sie entfaltet nicht nur gegen Botrytis und Rhizoctonia eine hervorragende fungizide Wirkung, sondern eignet sich auch zur erfolgreichen

Bekämpfung von Erysiphe- und Venturia-Species. Ueberdies wirken die Verbindungen systemisch. Darüber hinaus lassen sich Verbindungen der Formel I erfolgreich zum Schutz verderblicher Waren pflanzlicher Herkunft einsetzen. Sie bekämpfen Schimmelpilze wie Penicillium, Aspergillus, Rhizopus, Fusarium, Helminthosporium, Nigrospora und Alternaria sowie Bakterien wie Buttersäurebakterien und Hefen wie Candida. Diese Wirksubstanzen zeigen ferner hervorragende Wirkung gegen Boden- und Samen-bürtige Pilze.

Als Pflanzenschutzmittel besitzen die Verbindungen der Formel I ein für die praktische Anwendung in der Landwirtschaft sehr günstiges Wirkungsspektrum zum Schutze von Kulturpflanzen, ohne diese durch unerwünschte Nebenwirkungen nachteilig zu beeinflussen.

Die Wirkstoffe der Formel I können ferner auch als Beizmittel zur Behandlung von Saatgut (Früchte, Knollen, Körner) und Pflanzenstecklingen zum Schutz vor Pilzinfektionen sowie gegen im Erdboden auftretende phytopathogene Pilze eingesetzt werden, wobei sie sich insbesondere als Getreidebeizmittel in der Bekämpfung von Pilzorganismen, wie beispielsweise Fusarium-, Helminthosporium und Tilletia-Arten, auszeichnen.

Die Erfindung betrifft somit auch mikrobizide Mittel sowie die Verwendung der Verbindungen der Formel I zur Bekämpfung phytopathogener Mikroorganismen, insbesondere pflanzenschädigender Pilze bzw. die präventive Verhütung eines Befalls an Pflanzen und an Vorräten pflanzlicher Herkunft.

Als Zielkulturen für den Pflanzenschutz gelten im Rahmen dieser Erfindung beispielsweise folgende Pflanzenarten: Getreide: (Weizen, Gerste, Roggen, Hafer, Reis, Sorghum und Verwandte); Rüben: (Zucker- und Futterrüben): Kern-, Stein- und Beerenobst: (Aepfel, Birnen, Pflaumen, Pfirsiche, Mandeln, Kirschen, Erd-, Him- und Brombeeren); Hülsenfrüchte: (Bohnen, Linsen, Erbsen, Soja): Oelkulturen: (Raps, Senf, Mohn, Oliven, Sonnenblumen, Kokos, Rizinus, Kakao, Erdnüssen); Gurkengewächse: (Kürbis, Gurken, Melonen); Fasergewächse: (Baumwolle, Flachs, Hanf, Jute); Citrusfrüchte: (Orangen, Zitronen, Pampelmusen, Mandarinen); Gemüsesorten: (Spinat, Kopfsalat, Spargel, Kohlarten, Möhren, Zwiebeln, Tomaten, Kartoffeln, Paprika); Lorbeergewächse: (Avocado, Cinnamonum, Kampfer) oder Pflanzen wie Mais, Tabak, Nüsse, Kaffee, Zuckerrohr, Tee, Weinreben, Hopfen, Bananen-und Naturkautschukgewächse sowie Zierpflanzen (Compositen).

Im Vorratschutz werden die Verbindungen der Formel I in unveränderter Form oder vorzugsweise zusammen mit den in der Formulierungstechnik üblichen Hilfsmitteln eingesetzt und werden z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Enkapsulierung in z.B. polymeren Stoffen in bekannter Weise verarbeitet. Die Anwendungsverfahren wie Versprühen, Vernebeln, Verstäuben, Verstreuen, Bestreichen oder Giessen und die Form des Mittels werden den angestrebten Zielen und den gegebenen Verhältnissen angepasst. Günstige Aufwandmengen liegen im allgemeinen bei 0,01 bis höchstens 2 kg Aktivsubstanz pro 100 kg zu schützendes Gut; sie hängen jedoch ganz wesentlich von der Beschaffenheit (Grösse der Oberfläche, Konsistenz, Feuchtigkeitsgehalt) des Gutes und dessen Umgebungseinflüssen ab.

Unter Lager- und Vorratsgütern sollen im Rahmen vorliegender Erfindung pflanzliche Naturstoffe und deren Weiterverarbeitungsprodukte verstanden werden, beispielsweise die nachfolgend aufgezählten und aus dem natürlichen Lebenszyklus herausgenommenen Pflanzen, deren Pflanzenteile (Stengel, Blätter, Knollen, Samen, Früchte, Körner), die im frisch geernteten Zustand oder in weiterverarbeitbarer Form vorliegen (vorgetrocknet, befeuchtet, zerkleinert, gemahlen, geröstet). Als Beispiele, die keinen das Anwendungsgebiet limitierenden Charakter im Rahmen dieser Erfindung besitzen, seien folgende Agrarprodukte genannt: Getreide (wie Weizen, Gerste, Roggen, Hafer, Reis, Sorghum und Verwandte); Rüben (wie Möhren, Zucker- und Futterrüben); Kern-, Stein- und Beerenobst (wie Aepfel, Birnen, Pflaumen, Pfirsiche, Mandeln, Kirschen, Erd-, Him- und Brombeeren); Hülsenfrüchte (wie Bohnen, Linsen, Erbsen, Soja); Oelkulturen (wie Raps, Senf, Mohn, Oliven, Sonnenblumen, Kokos, Rizinus, Kakao, Erdnüsse); Gurkengewächse (wie Kürbis, Gurken, Melonen); Fasergewächse (wie Baumwolle, Flachs, Hanf, Jute, Nessel); Citrusfrüchte; Gemüsesorten (wie Spinat, Salat, Spargel, Kohlarten, Zwiebeln, Tomaten, Kartoffeln, Paprika): Lorbeergewächse (wie Avocadom Cinnamonum, Kampfer) oder Mais, Tabak, Nüsse, Kaffee, Zuckerrohr, Tee, Weintrauben, Kastanien, Hopfen, Bananen, Gras und Heu.

Ein bevorzugtes Verfahren zum Aufbringen des Wirkstoffes besteht in einem Besprühen oder Benetzen des Substrats mit einer flüssigen Aufbereitung oder im Vermischen des Substrats mit einer festen Aufbereitung der Aktivsubstanz. Das beschriebene Konservierungs-Verfahren ist ein Teil der vorliegenden Erfindung.

Wirkstoffe der Formel I werden üblicherweise in Form von Zusammensetzungen verwendet und können gleichzeitig oder nacheinander mit weiteren Wirkstoffen auf die zu behandelnde Fläche, Pflanze oder Substrat gegeben werden. Diese weiteren Wirkstoffe können sowohl Düngemittel, Spurenelement-Vermittler oder andere das Pflanzenwachstum beeinflussende Präparate sein. Es können aber auch selektive Herbizide, Insektizide, Fungizide, Bakterizide, Nematizide, Molluskizide oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln. Besonders vorteilhafte Zusatzstoffe sind Phospholipide.

Ein bevorzugtes Verfahren zum Aufbringen eines Wirkstoffes der Formel I bzw. eines (agro)chemischen Mittels, das mindestens einen dieser Wirkstoffe enthält, ist das Aufbringen auf das Blattwerk (Blattapplikation). Anzahl der Applikationen und Aufwandmenge richten sich dabei nach dem Befallsdruck für den entsprechenden Erreger (Pilzsorte). Die Wirkstoffe der Formel I können aber auch über den Erdboden durch das Wurzelwerk in die Pflanze gelangen (systemische Wirkung), indem man den Standort der Pflanze mit einer flüssigen Zübereitung tränkt oder die Substanzen in fester Form in den Boden einbringt, z.B. in Form von Granulat (Bodenapplikation). Die Verbindungen der Formel I können aber auch auf Samenkörner aufgebracht werden (Coating), indem man die Körner entweder mit einer flüssigen Zübereitung des Wirkstoffs tränkt oder sie mit einer festen Zübereitung beschichtet. Darüber hinaus sind in besonderen Fällen weitere Applikationsarten möglich, so z.B. die gezielte Behandlung der Pflanzenstengel oder der Knospen.

Die Verbindungen der Formel I werden dabei in unveränderter Form oder vorzugsweise zusammen mit den in der Formulierungstechnik üblichen Hilfsmittel eingesetzt und werden daher z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 50 g bis 5 kg Aktivsubstanz (AS) je ha; bevorzugt 100 g bis 2 kg AS/ha, insbesondere bei 200 g bis 600 g AS/ha.

Die Formulierungen d.h. die den Wirkstoff der Formel I und gegebenenfalls einen festen oder flüssigen Zusatzstoff enthaltenden Mittel, Zübereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder -ethylether, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl, Sonnenblumenöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht adsorptive Trägermaterialien z.B. Calcit oder Sand in Frage. Darüber hinaus kann eine Vielzahl von vorgranulierten Materialien anorganischer oder organischer Natur wie insbesondere Dolomit oder zerkleinerte Pflanzenrückstände, wie z.B. Korkmehl oder Sägemehl, verwendet werden.

Als oberflächenaktive Verbindungen kommen je nach Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z.B. die Na-oder K-Salze der Oel- oder Stearinsäure, oder von natürlichen Fettsäuregemischen, die z.B. aus Kokosnuss- oder Talgöl gewonnen werden können. Ferner sind auch die Fettsäure-methyllaurinsalze zu erwähnen.

Häufiger werden jedoch sog. synthetische Tenside verwendet, insbesondere Fettsulfonate, Fettsulfate, sulfonierte Benzimidazolderivate oder Alkylsulfonate.

Die Fettsulfonate oder -sulfate liegen in der Regel als Alkali-, Erdalkali- oder gegebenenfalls substituierte Ammoniumsalze vor und weisen einen Alkalirest mit 8 bis 22 C-Atomen auf, wobei Alkyl auch den

Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxid-Addukten. Die sulfonierten Benzimidazolderivate enthalten vorzugsweise 2-Sulfonsäuregruppen und einen Fettsäurerest mit 8-22 C-Atomen. Alkylarylsulfonate sind z.B. die Na-, Ca- oder Triethanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureessters eines p-Nonylphenol-(4-14)-ethylenoxidaddukts in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Ethylenglykolethergruppen und 10 bis 100 Propylenglykolethergruppen enthaltenden Polyethylenoxidaddukte an Polypropylenglykol, Ethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyglykolether, Polypropylen-Polyethylenoxidaddukte, Tributylphenoxypolyethylenethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)ethylammoniumbromid. Auf dem Vorratsschutzgebiet werden die für die menschliche und tierische Ernährung unbedenklichen Zuschlagstoffe bevorzugt.

Die agrochemischen Zübereitungen enthalten in der Regel 0,1 bis 99 Gew.%, insbesondere 0,1 bis 95 Gew.%, Wirkstoff der Formel I, 99,9 bis 1 Gew.%, insbesondere 99,8 bis 5 Gew.%, eines festen oder flüssigen Zusatzstoffes und 0 bis 25 Gew.%, insbesondere 0,1 bis 25 Gew.%, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Derartige (agro)chemische Mittel sind ein Bestandteil der vorliegenden Erfindung.

Die nachfolgenden Beispiele dienen der Illustration der Erfindung, ohne dieselbe einzuschränken (Prozente und Teile beziehen sich auf Gewicht; Temperaturen sind in Celsiusgraden angegeben).

1. Herstellungsbeispiele:

1.1. Herstellung von 2-[3-(2,2-Difluorbenzodioxol-4-yl)-4-cyanopyrrol-1-yl]-essigsäuremethylester. (Zwischenprodukt)

Zu 15 g 3-(2,2-Difluorbenzodioxol-4-yl)-4-cyanopyrrol, gelöst in 20 ml Dimethylformamid werden 9,1 g Kaliumcarbonat zugegeben. Zu dieser Suspension tropft man bei 20 bis 30 °C 6,1 ml Bromessigsäuremethylester zu und erhitzt während 18 Stunden auf 55 °C. Das Reaktionsgut wird im Rotationsverdampfer eingedampft und der Rückstand zwischen je 100 ml Wasser und Methylenchlorid verteilt. Die abgetrennte organische Phase wird mit gesättigter Kochsalzlösung gewaschen, über Natriumsulfat getrocknet, filtriert und das Lösungsmittel wird verdampft. Man erhält 2-[3-(2,2-Difluorbenzodioxol-4-yl)-4-cyanopyrrol-1yl]-essigsäuremethylester als beige-farbene Kristalle mit einem Schmelzpunkt von 138-140 °C.

1.2 Herstellung von 3-Hydroxy-2-[3-(2,2-Difluorbenzodioxol-4-yl)-4-cyanopyrrol-1-yl]-acrylsäuremethylester.

Zu 3,14 g 2-[3-(2,2-Difluorbenzodioxol-4-yl)-4-cyanopyrrol-lyl]-essigsäuremethylester, gelöst in 40 ml Ameisensäuremethylester werden bei 5-10° C 0,36 g 80 %iges Natriumhydrid zugegeben. Das Reaktionsgemisch wird über Nacht bei Raumtemperatur gerührt, mit 1 N Salzsäure sauergestellt mit Aether extrahiert und der Extrakt eingedampft. Das Rohgemisch wird mit Toluol: Essigester = 6:1 an Kieselgel chromatographiert. Man erhält hellbeige-farbene Kristalle vom Schmelzpunkt 141-143° C.

In analoger Weise werden die in der Tabelle angegebenen Verbindungen 2 bis 15 hergestellt.

1.3 Herstellung des Na-Salzes von 3-Hydroxy-2-[3-(2,2-Difluorbenzodioxol-4-yl)-4-cyanopyrrol-1-yl]-acrylsäuremethylester.

Zu 3,14 g 2-[3-2,2-Difluorbenzodioxol-4-yl)-4-cyanopyrrol-lyl]-essigsäuremethylester, gelöst in 40 ml Ameisensäuremethylester werden bei 5-10° C 0,36 g 80 %iges Natriumhydrid zugegeben. Das Reaktionsgemisch wird über Nacht bei Raumtemperatur gerührt, abfiltriert, das Kristallisat mit Ether gewaschen und getrocknet. Das Zielprodukt schmilzt höher als 210° C (Zers.).

Tabelle 1

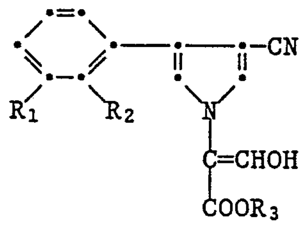

| Bsp. Nr. | $R^1$ | $R^2$ | $R^3$ | Smp. |
|---|---|---|---|---|
| 1 | 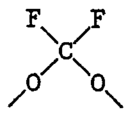 | | $CH_3$ | 141-143°C |
| 2 | H | H | $CH_3$ | 120-140°C |
| 3 | Cl | H | $CH_3$ | 122-123°C |
| 4 | H | Cl | $CH_3$ | 122-130°C |
| 5 | Cl | Cl | $CH_3$ | 153-155°C |
| 6 | H | Br | $CH_3$ | 108-110°C |
| 7 | H | $CF_3$ | $CH_3$ | 114-116°C |
| 8 | H | $CH_3$ | $CH_3$ | |
| 9 | 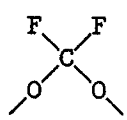 | | $C_2H_5$ | 105-107°C |
| 10 | 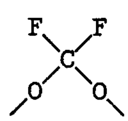 | | $n-C_6H_{13}$ | |
| 11 | 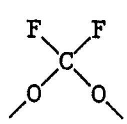 | | 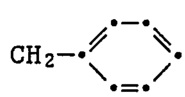 | |

Tabelle 1 (Fortsetzung)

| Bsp. Nr. | $R^1$ | $R^2$ | $R^3$ | Smp. |
|---|---|---|---|---|
| 12 | H | $NO_2$ | $CH_3$ | |
| 13 | Cl | $NO_2$ | $CH_3$ | |
| 14 | 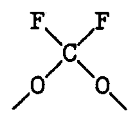 | | 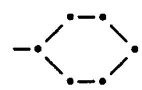 | |
| 15 | 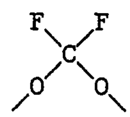 | | 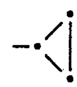 | |

2. Formulierungsbeispiele für flüssige Wirkstoffe der Formel I (% = Gewichtsprozent)

| 2.1. Emulsion-Konzentrate | a) | b) | c) |
|---|---|---|---|
| Wirkstoff aus Tabelle 1 | 25% | 40% | 50% |
| Ca-Dodecylbenzolsulfonat | 5% | 8% | 6% |
| Ricinusöl-polyäthylenglykoläther (36 Mol Aethylenoxid) | 5% | - | - |
| Tributylphenoyl-polyäthylenglykoläther (30 Mol Aethylenoxid) | - | 12% | 4% |
| Cyclohexanon | - | 15% | 20% |
| Xylolgemisch | 65% | 25% | 20% |

Aus solchen Konzentraten können durch Verdünnen mit Wasser Emulsionen jeder gewünschten Konzentration hergestellt werden.

| 2.2. Lösungen | a) | b) | c) | d) |
|---|---|---|---|---|
| Wirkstoff aus Tabelle 1 | 80% | 10% | 5% | 95% |
| Aethylenglykol-monomethyläther | 20% | - | - | - |
| Polyäthylenglykol M G 400 | - | 70% | - | - |
| N-Methyl-2-pyrrolidon | - | 20% | - | - |
| Epoxydiertes Kokosnussöl | - | - | 1% | 5% |
| Benzin (Siedegrenzen 160-190 °C) | - | - | 94% | - |
| (MG = Molekulargewicht) | | | | |

| 2.3. Granulate | a) | b) |
|---|---|---|
| Wirkstoff aus Tabelle 1 | 5% | 10% |
| Kaolin | 94% | |
| Hochdisperse Kieselsäure | 1% | - |
| Attapulgit | - | 90% |

Der Wirkstoff wird in Methylenchlorid gelöst, auf den Träger aufgesprüht und das Lösungsmittel anschliessend im Vakuum abgedampft.

| 2.4. Stäubemittel | a) | b) |
|---|---|---|
| Wirkstoff aus Tabelle 1 | 2% | 5% |
| Hochdisperse Kieselsäure | 1% | 5% |
| Talkum | 97% | - |
| Kaolin | - | 90% |

Durch inniges Vermischen auf Trägerstoffe mit dem Wirkstoff erhält man gebrauchsfertige Stäubemittel.

| 2.5. Spritzpulver | a) | b) | c) |
|---|---|---|---|
| Wirkstoff aus Tabelle 1 | 25% | 50% | 75% |
| Na-Ligninsulfonat | 5% | 5% | - |
| Na-Laurylsulfat | 3% | - | 5% |
| Na-Diisobutylnaphthalinsulfonat | - | 6% | 10% |
| Octylphenolpolyäthylenglykoläther (7-8 Mol Aethylenoxid) | - | 2% | - |
| Hochdisperse Kieselsäure | 5% | 10% | 10% |
| Kaolin | 62% | 27% | - |

Der Wirkstoff wird mit den Zusatzstoffen gut vermischt und in einer geeigneten Mühle vermahlen. Man erhält Spritzpulver, die sich mit Wasser zu Suspensionen jeder gewünschten Konzentration verdünnen lassen.

| 2.6. Emulsions-Konzentrat | |
|---|---|
| Wirkstoff aus Tabelle 1 | 10% |
| Octylphenolpolyäthylenglykoläther (4-5 Mol Aethylenoxid) | 3% |
| Ca-Dodecylbenzolsulfonat | 3% |
| Ricinusölpolyglykoläther (35 Mol Aethylenoxid) | 4% |
| Cyclohexanon | 30% |
| Xylolgemisch | 50% |

Aus diesem Konzentrat können durch Verdünnen mit Wasser Emulsionen jeder gewünschten Konzentration hergestellt werden.

| 2.4. Stäubemittel | a) | b) |
|---|---|---|
| Wirkstoff aus Tabelle 1 | 5% | 8% |
| Talkum | 95% | - |
| Kaolin | - | 92% |

Man erhält anwendungsfertige Stäubemittel, indem der Wirkstoff mit dem Träger vermischt auf einer geeigneten Mühle vermahlen wird.

| 2.8. Extruder Granulat | |
|---|---|
| Wirkstoff aus Tabelle 1 | 10% |
| Na-Ligninsulfonat | 2% |
| Carboxymethylcellulose | 1% |
| Kaolin | 87% |

Der Wirkstoff wird mit den Zusatzstoffen vermischt, vermahlen und mit Wasser angefeuchtet. Dieses Gemisch wurd extrudiert und anschliessend im Luftstrom getrocknet.

| 2.9. Umhüllungs-Granulat | |
|---|---|
| Wirkstoff aus Tabelle 1 | 3% |
| Polyäthylenglykol M G 200 | 3% |
| Kaolin | 94% |
| (MG = Molekulargewicht) | |

3. Biologische Beispiele:

Beispiel 3.1.: Wirkung gegen Venturia inaequalis auf Apfel Residualprotektive Wirkung

Apfelsämlinge mit ca. 5 entwickelten Blättern wurden mit einer aus Spritzpulver, nach einem der obigen Beispiele hergestellten Spritzbrühe (0,02 % Aktivsubstanz aus der Tabelle 1) gleichmässig besprüht. Nach 24 Stunden wurden die behandelten Pflanzen mit einer Konidiensuspension des Pilzes infiziert. Die Pflanzen wurden dann während 5 Tagen bei 90-100 % relativer Luftfeuchtigkeit inkubiert und während 10 weiteren Tagen in einem Gewächshaus bei 20-24 °C aufgestellt. Der Schorfbefall wurde 15 Tage nach der Infektion beurteilt, dabei hemmten Spritzbrühen, die als Aktivsubstanz eine der Verbindungen aus der Tabelle 1 (z.B. Verbindung Nr. 1 oder 5) enthielten, den Pilzbefall deutlich.

Beispiel 3.2.: Wirkung gegen Botrytis cinerea auf Bohnen Residual protektive Wirkung

Ca. 10 cm hohe Bohnen-Pflanzen werden mit einer aus Spritzpulver des Wirkstoffes hergestellten Spritzbrühe (0,02 % Aktivsubstanz) besprüht. Nach 48 Stunden werden die behandelten Pflanzen mit einer Konidiensuspension des Pilzes infiziert. Nach einer Inkubation der infizierten Pflanzen während 3 Tagen bei 95 - 100 % relativer Luftfeuchtigkeit und 21 °C erfolgt die Beurteilung des Pilzbefalls. Die Verbindungen aus der Tabelle 1 hemmten in vielen Fällen die Pilzinfektion sehr stark. Bei einer Konzentration von 0.02 % erwiesen sich z.B. die Verbindungen Nr. 1 oder 5 als voll wirksam (Krankheitsbefall 0 bis 5 %). Befall unbehandelter aber infizierter Bohnenpflanzen betrug 100 %.

Beispiel 3.3.: Wirkung gegen Botrytis cinerea an Apfelfrüchten

Künstlich verletzte Aepfel werden behandelt, indem eine aus Spritzpulver der Wirksubstanz hergestellte Spritzbrühe von 6 ppm Konzentration auf die Verletzungsstellen aufgetropft wird. Die behandelten Früchte werden anschliessend mit einer Sporensuspension von Botrytis cinerea inokuliert und während einer Woche bei einer hohen Luftfeuchtigkeit und ca. 20 °C inkubiert.

Bei der Auswertung werden die angefaulten Verletzungsstellen gezählt und daraus die fungizide Wirkung der Testsubstanz abgeleitet. Verbindungen aus Tabelle 1 waren gut wirksam gegen Botrytis-Befall

von Apfelfrüchten. Unter anderen hemmten die Verbindungen Nr. 1 und 5 den Pilzbefall im Vergleich zu unbehandelten Kontrollfrüchten (mit 100 % Befall) fast vollständig.

Beispiel 3.4.: Wirkung gegen Alternaria solani auf Tomate

Tomatenpflanzen werden nach dreiwöchiger Anzucht mit einer aus Spritzpulver des Wirkstoffs hergestellten Spritzbrühe (0,006 % Aktivsubstanz) besprüht. Nach 24 Stunden werden die Tomatenpflanzen mit einer Konidiensuspension des Pilzes infiziert. Die Beurteilung der fungiziden Wirkung erfolgt aufgrund des Pilzbefalls nach einer Inkubation der infizierten Pflanzen während 8 Tagen bei hoher Luftfeuchtigkeit und einer Temperatur von 18 - 22° C. Verbindungen aus der Tabelle 1 bewirkten eine starke Reduktion des Alternariabefalls; so hemmte die Substanz Nr. 1 den Befall vollständig (0 - 5%).

Beispiel 3.5.: Wirkung gegen Pyricularia oryzae auf Reis

Reispflanzen werden nach zweiwöchiger Anzucht mit einer aus Spritzpulver des Wirkstoffes hergestellten Spritzbrühe (0,02 % Aktivsubstanz) besprüht. Nach 48 Stunden werden die behandelten Pflanzen mit einer Konidiensuspension des Pilzes infiziert. Nach 5 Tagen Inkubation bei 95 - 100 % relativer Luftfeuchtigkeit und 24° C wird der Pilzbefall beurteilt. Verbindungen aus Tabelle 1 zeigten eine gute Hemmung des Pyriculariabefalls, so reduzierte z.B. die Verbindung Nr. 1 den Befall auf weniger als 10 %.

Beispiel 3.6.: Wirkung gegen Rhizoctonia solani (Bodenpilz auf Reispflanzen)

Protektiv-lokale Blattapplikation

12 Tage alte Reispflanzen werden mit einer aus einer Formulierung der Wirkstoffe hergestellten Spritzbrühen (200 und 60 ppM) besprüht. Ein Tag später werden die behandelten Pflanzen mit einer Suspension von Myzel und Sklerotien von R. solani infiziert. Nach 6-tägiger Inkubation bei 27° C (Tag), bzw. 23° C (Nacht) und 100 % rel. Luftfeuchtigkeit (Feuchtkasten) in der Klimakammer wird der Pilzbefall auf Blattscheide, Blättern und Stengel beurteilt.

Verbindungen aus den Tabellen zeigen gute Wirkung durch Hemmung des Rhizoctonia-Befalls. Unbehandelte jedoch infizierte Kontrollpflanzen weisen dagegen einen Befall von 100 % auf. Der Pilzbefall wird beispielsweise durch die Verbindung Nr. 1 bis auf 0-5, durch die Verbindung Nr. 5 bis auf 5-20 % gehemmt.

## Ansprüche

1. Verbindungen der Formel I

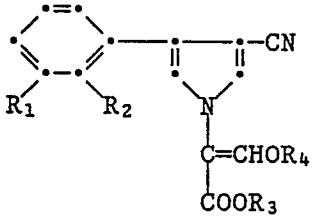

(I),

in welcher

$R_1$ und $R_2$ voneinander unabhängig unsubstituiertes oder halogensubstituiertes $C_1$-$C_8$-Alkyl, Halogen, Nitro oder Wasserstoff, oder $R_1$ und $R_2$ zusammen $-OCF_2O-$,

$R_3$ $C_1$-$C_8$-Alkyl, Cycloalkyl oder Benzyl und

$R_4$ Wasserstoff oder ein Alkali- oder Erdalkalimetall bedeutet.

2. Verbindungen der Formel I, Anspruch 1, in welchen $R_4$ Wasserstoff bedeutet.

3. Verbindungen der Formel I, Anspruch 2, in welchen
$R_1$ und $R_2$ voneinander unabhängig unsubstituiertes oder halogensubstituiertes $C_1$-$C_6$-Alkyl, Halogen, Nitro oder Wasserstoff, oder $R_1$ und $R_2$ zusammen $-OCF_2O-$ und
$R_3$ $C_1$-$C_6$-Alkyl oder Benzyl bedeuten.

4. Verbindungen gemäss Anspruch 3, in welchen
$R_1$ und $R_2$ voneinander unabhängig Methyl, Trifluormethyl, Chlor, Brom oder Wasserstoff, oder $R_1$ und $R_2$ zusammen $-OCF_2O-$ und
$R_3$ Methyl, Aethyl, n-Hexyl oder Benzyl bedeuten.

5. Verbindungen gemäss Anspruch 4, in welchen $R_3$ Methyl oder Aethyl bedeuten.

6. 2-[3-(2,2-Difluorbenzodioxol-4-yl)-4-cyanopyrrol-1-yl]-3-hydroxyacrylsäuremethylester gemäss Anspruch 5.

7. 2-[3-(2,3-Dichlorphenyl)-4-cyanopyrrol-1-yl]-3-hydroxyacrylsäuremethylester gemäss Anspruch 5.

8. Verfahren zur Herstellung der Verbindungen der Formel I, Anspruch 1, dadurch gekennzeichnet, dass man eine Verbindung der Formel II in Gegenwart einer Base mit einer Verbindung der Formel III oder mit einem anderen Formylierungsreagens

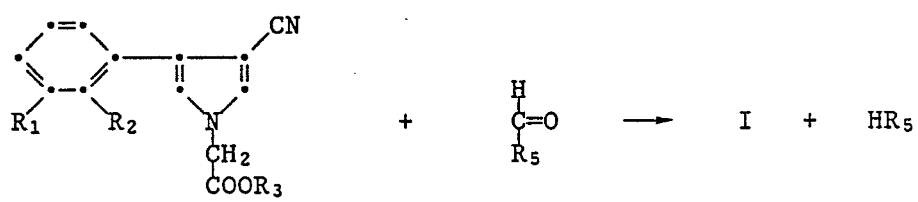

II III

umsetzt,
wobei die Reste $R_1$ - $R_3$ die unter Formel I angegebene Bedeutung haben und
$R_5$ einen Alkoxy- oder einen Dialkylaminorest bedeutet.

9. Mikrobizides Mittel zur Bekämpfung oder Verhütung eines Befalls von lebenden Pflanzen und/oder zur Konservierung von verderblichem Lagergut pflanzlicher Herkunft, dadurch gekennzeichnet, dass es als mindestens eine aktive Komponente eine der in Anspruch 1 definierten Verbindungen enthält.

10. Mittel nach Anspruch 9, dadurch gekennzeichnet, dass es 0,1 bis 99 % eines Wirkstoffs der Formel I, 99,9 bis 1 % eines festen oder flüssigen Zusatzstoffes und 0 bis 25 % eines Tensides enthält.

11. Mittel nach Anspruch 10, dadurch gekennzeichnet, dass es 0,1 bis 95 % eines Wirkstoffes der Formel I 99,8 bis 5 % eines festen oder flüssigen Zusatzstoffes und 0,1 bis 25 % eines Tensides enthält.

12. Verfahren zur Herstellung eines in den Ansprüchen 9 bis 11 definierten (agro)chemischen Mittels, dadurch gekennzeichnet, dass man mindestens eine Verbindung der Formel I gemäss einem der Ansprüche 1 bis 7 mit geeigneten festen oder flüssigen Zusatzstoffen und Tensiden innig vermischt.

13. Verfahren zur Bekämpfung oder Verhütung eines Befalls von Kulturpflanzen durch phytopathogene Mikroorganismen, dadurch gekennzeichnet, dass man eine Verbindung der Formel I gemäss einem der Ansprüche 1 bis 7 auf die Pflanze, Pflanzenteile oder deren Standort appliziert.

14. Verfahren zur Beizung von Saatgut und Pflanzenstecklingen zum Schutz gegen den Befall durch Pilzorganismen, dadurch gekennzeichnet, dass man eine Verbindung der Formel I gemäss einem der Ansprüche 1 bis 7 auf das Saatgut oder die Pflanzenstecklinge appliziert.

15. Verfahren zur Konservierung bzw. zum Schutz von Vorrats- oder Lagergut pflanzlicher Herkunft vor schädlichen Mikroorganismen durch Behandlung des Gutes mit mindestens einer mikrobizid wirksamen Menge einer Verbindung der Formel I gemäss einem der Ansprüche 1 bis 7.

16. Verwendung von Verbindungen der Formel I gemäss Anspruch 1 zur Bekämpfung und/oder zur präventiven Verhütung eines Befalls von Mikroorganismen.

17. Verwendung gemäss Anspruch 16 dadurch gekennzeichnet, dass es sich bei den Mikroorganismen um phytopathogene Pilze handelt.

18. Verwendung gmeäss Anspruch 17 gegen Pilze aus den Klassen Ascomycetes, Basidiomycetes oder Fungi imperfecti.

19. Verwendung gemäss Anspruch 18 gegen Botrytis-Pilze.

Patentansprüche für folgenden Vertragsstaat: ES

1. Verfahren zur Herstellung der Verbindungen der Formel I

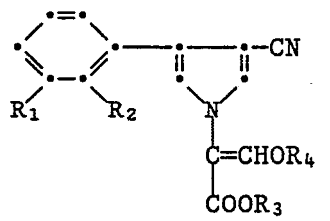

(I),

in welcher
$R_1$ und $R_2$ voneinander unabhängig unsubstituiertes oder halogensubstituiertes $C_1$-$C_8$-Alkyl, Halogen, Nitro oder Wasserstoff, oder $R_1$ und $R_2$ zusammen $-OCF_2O-$,
$R_3$ $C_1$-$C_8$-Alkyl, Cycloalkyl oder Benzyl und
$R_4$ Wasserstoff oder ein Alkali- oder Erdalkalimetall bedeutet,
dadurch gekennzeichnet, dass man eine Verbindung der Formel II in Gegenwart einer Base mit einer Verbindung der Formel III oder mit einem anderen Formylierungsreagens

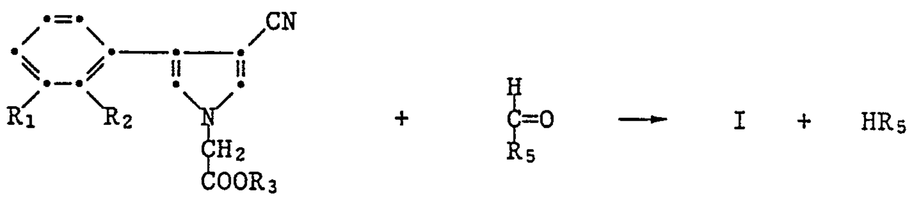

II III

umsetzt,
wobei $R_5$ einen Alkoxy- oder einen Dialkylaminorest bedeutet.

2. Verfahren gemäss Anspruch 1, dadurch gekennzeichnet, dass $R_4$ Wasserstoff bedeutet.

3. Verfahren gemäss Anspruch 2, dadurch gekennzeichnet, dass $R_1$ und $R_2$ voneinander unabhängig unsubstituiertes oder halogensubstituiertes $C_1$-$C_6$-Alkyl, Halogen, Nitro oder Wasserstoff, oder $R_1$ und $R_2$ zusammen $-OCF_2O-$ und
$R_3$ $C_1$-$C_6$-Alkyl oder Benzyl bedeuten.

4. Verfahren gemäss Anspruch 3, dadurch gekennzeichnet, dass
$R_1$ und $R_2$ voneinander unabhängig Methyl, Trifluormethyl, Chlor, Brom oder Wasserstoff, oder $R_1$ und $R_2$ zusammen $-OCF_2O-$ und
$R_3$ Methyl, Aethyl, n-Hexyl oder Benzyl bedeuten.

5. Verfahren gemäss Anspruch 4, dadurch gekennzeichnet, dass $R_3$ Methyl oder Aethyl bedeuten.

6. Verfahren zur Herstellung des 2-[3-( 2,2-Difluorbenzodioxol-4-yl)-4-cyanopyrrol-1-yl]-3-hydroxyacrylsäuremethylesters gemäss Anspruch 5.

7. Verfahren zur Herstellung des 2-[3-(2,3-Dichlorphenyl)-4-cyanopyrrol-1-yl]-3-hydroxyacrylsäuremethylesters gemäss Anspruch 5.

Europäisches Patentamt

# EUROPÄISCHER RECHERCHENBERICHT

Nummer der Anmeldung

EP 90 10 4215

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A,D | EP-A-0 182 737 (CIBA-GEIGY AG) * Ansprüche 1,8,15-18 * --- | 1,9-19 | C 07 D 207/34<br>C 07 D 405/04<br>A 01 N 43/36 |
| A | DE-A-2 927 480 (NIPPON SODA CO LTD) * Ansprüche 1,6 * --- | 1,9-19 | |
| A | EP-A-0 206 523 (IMPERIAL CHEMICAL INDUSTRIES PLC) * Ansprüche 1,8,11,12 * ----- | 1,8-19 | |

RECHERCHIERTE SACHGEBIETE (Int. Cl.5)

C 07 D 207/00
A 01 N 43/00

Der vorliegende Recherchenbericht wurde für alle Patentansprüche erstellt

| Recherchenort | Abschlußdatum der Recherche | Prüfer |
|---|---|---|
| BERLIN | 11-06-1990 | VAN AMSTERDAM L.J.P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)